(19) Europäisches Patentamt
European Patent Office
Office européen des brevets

(11) Publication number: **0 151 964**
**A2**

(12) **EUROPEAN PATENT APPLICATION**

(21) Application number: 85100564.5

(22) Date of filing: 20.01.85

(51) Int. Cl.[4]: C 07 D 307/32
A 61 K 31/365

(30) Priority: 02.02.84 IT 1939084

(43) Date of publication of application:
21.08.85 Bulletin 85/34

(84) Designated Contracting States:
AT BE CH DE FR GB IT LI LU NL SE

(71) Applicant: Laboratorio Farmaceutico C.T. s.r.l.
Via Dante Alighieri, 71
I-18038 Sanremo(IT)

(72) Inventor: Tessitore, Pietro Tomaso
Via Dante Alighieri, 71
I-18038 Sanremo(IT)

(74) Representative: Melocchi, Paola
Via Ulisse Salis 28
I-20161 Milano(IT)

(54) **New derivatives of alpha-amino-gamma-butyrolactone, method for the preparation thereof and pharmaceutical compositions containing them.**

(57) The present invention relates to new derivatives of α-amino-γ-butyrolactone and their pharmaceutically acceptable salts, endowed with anti-convulsant, anti-epileptic, sedative action and able to inhibit the ingestion of alcohol and intoxicating drinks.

The invention relates also to the method for preparing the derivatives of the invention as well as the pharmaceutical compositions containing them.

EP 0 151 964 A2

The present invention relates to new derivatives of $\alpha$-amino-$\gamma$-butyrolactone, to the method for the preparation thereof and to the pharmaceutical compositions containing them.

Namely, the present invention relates to derivatives of $\alpha$-amino-$\gamma$-butyrolactone comprised in the general formula (I):

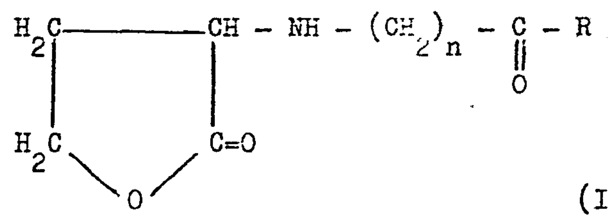

(I)

(wherein R is a linear or branched alkyl radical containing from 4 to 8 carbon atoms; or R is -OR', R' being a linear or branched alkyl radical containing from 1 to 5 C; <u>n</u> is always zero when R is alkyl, or <u>n</u> is an integer different from zero and comprised between 1 and 5 when R is -OR') and their pharmaceutically acceptable salts.

The derivatives of $\alpha$-amino-$\gamma$-butyrolactone of the present invention are endowed with anti-convulsant, anti-epileptic and sedative action when administered to mammals and furthermore they are able to inhibit drinking of alcohol and intoxicating drinks.

In a previous Italian Patent Application filed in name of Laboratorio Farmaceutico C.T. ( It. Pat. Appln. 23806 A/83) there were described and claimed derivatives of $\alpha$-alkylamino-$\gamma$-butyrolactone and the pharmaceutically acceptable salts thereof able to inhibit drinking of alcohol and intoxicating drinks.

Compounds comprised in the general formula (I) are new and not described in literature.

Among the compounds comprised in the general formula (I) wherein R is a linear or branched alkyl radical containing from 4 to 8 C and <u>n</u> is zero, the following compounds may be mentioned:

$\alpha$-n.butylcarbonyl-amino-$\gamma$-butyrolactone;

$\alpha$-isobutylcarbonyl-amino-$\gamma$-butyrolactone;

α-tert.butylcarbonyl-amino-γ-butyrolactone;

α-n.pentylcarbonyl-amino-γ-butyrolactone.

Among the above mentioned compounds, the exemplary compound according to the present invention is α-n.butylcarbonyl-amino-γ-butyrolactone ( which hereinafter will be indicated as PTT-01 for sake of brevity).

Among the compounds comprised in the general formula (I) wherein R is -OR' ( R' being a linear or branched alkyl radical containing from 1 to 5 C) and n is an integer different from zero and comprised betwen 1 and 5, the following compounds may be mentioned:

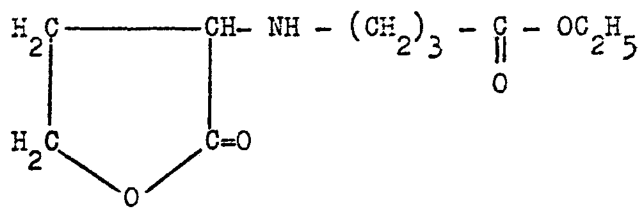

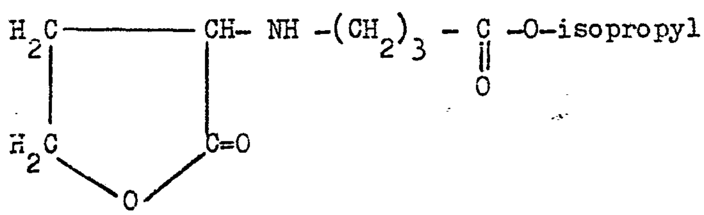

It is another object of the present invention to provide the method for preparing the compounds of the general formula (I).

The compounds comprised in the general formula (I) wherein R is a linear or branched alkyl radical containing from 4 to 8 C and n is always zero, are prepared by allowing the corresponding chloride of the acid to react with the selected α-amino-γ-butyrolactone, at reduced temperatures and in the presence of an organic base, for instance pyridine.

When in the general formula (I) R is -OR' ( R' being a linear or branched alkyl radical containing from 1 to 5 C) and n, diffe=

rent from zero, is an integer comprised between 1 and 5, the desi= red compounds are prepared from the desired esters of the ω -ami= noacids which are then allowed to react with α -halo-γ-butyrolac tone.

Experimental studies carried out with PTT-01 indicated that the compound produces a sedative effect, a slight hypnotic effect and an important anti-convulsant effect when administered to labo= ratory animals.

$LD_{50}$ of PTT-01 is so high that toxicity is nearly neglegible.

I. Acute toxicity in animals

PTT-01 was administered to adult rats ( Wistar-Morini Strain) of both sexes after fasting overnight. It was administered in an aqueous 1% suspension of tragacanth gum orally by gastric probe or intraperitoneally. The data in Table 1 show that:

(a) The $LD_{50}$ was more than 2g/kg by either route of administra tion.

(b) The highest oral dose produced a sedative effect, but it did not cause the loss of the righting reflex; on the other hand, the sedative effect was obtained starting from the lowest intrape= ritoneal dose ( a dose which was ineffective when given orally), and at 1 g/kg the loss of the righting reflex occurred; the dura= tion of this effect was proportional to the size of the administe= red dose.

(c) The sedative and hypnotic effects were not long lasting and the animal returned to normal.

TABLE 1- Acute toxicity of PTT-01 in rats

| Dose mg/kg | Dead*/ treated | Under hypnosis **/ treated | Remarks |
|---|---|---|---|
| Intraperitoneal | | | |
| 25 | 0/4 | 0/4 | Sedated for about 15 minutes |
| 50 | 0/4 | 0/4 | Sedated for about 20 minutes |
| 100 | 0/4 | 4/4 | Under hypnosis for about 15 minutes |
| 150 | 0/4 | 4/4 | Under hypnosis for about 30 minutes |
| 200 | 0/4 | 4/4 | Under hypnosis for about 60 minutes |
| Oral *** | | | |
| 25 | 0/4 | 0/4 | No particular effect |
| 50 | 0/4 | 0/4 | No particular effect |
| 100 | 0/4 | 0/4 | Slightly sedated |
| 150 | 0/4 | 0/4 | Sedated |
| 200 | 0/4 | 0/4 | Very sedated |

* Within 24 hours

** Loss of the righting reflex

*** By gastric probe

II. Subacute toxicity in animals

The acute toxicity studies showed that a dose of 50 mg/kg of PTT-01 by oral route did not cause any particular effect in rats, but a dose of 100 mg/kg produced a mild sedative effect. Accor= dingly, 8 rats were given 50 mg/kg daily for 6 consecutive days. No sedative effect or other abnormal symptoms were observed, show ing that there was no tendency for PTT-01 to accumulate in the or= ganism.

III. Sedative effect

The sedative effect of PTT-01 was demonstrated in the follow ing experimental models: (a) the enhancement of the hypnotic ef = fect of sodium pentobarbital in the mouse; (b) spontaneous mobili= ty in the rat; and (c) the ability of the mouse to remain on a rol ling bar.

(a) Enhancement of the hypnotic effect

40 mg/kg of sodium pentobarbital was injected intraperitoneal ly into mice and note was taken of animals losing the righting re= flex and for how long. Other mice received a preliminary treat= ment with PTT-01 at a dosage of 25 and 50 mg/kg, intraperitoneally and thereafter sodium pentobarbital was given. The hypnotic ef= fect of sodium pentobarbital was markedly enhanced and its enhance ment was greater with the 50 mg/kg dose of PTT-01 than with the 25 mg/kg dose. Results are given in Table 2.

(b) Spontaneous mobility

Several groups of 4 mice each were placed in an apparatus de signed to provide a measure of spontaneous mobility by counting the number of interruptions of a light beam for a period of 10 mi= nutes. After preliminary measurement of mobility, animals were injected with PTT-01 (25 and 50 mg/kg) intraperitoneally. Con= trols received saline solution. Then minutes thereafter the mea=

surement of mobility was repeated. Five groups of mice were used as controls and 6 other groups were treated with varying doses of PTT-01. Table 3 shows that mobility had fallen to 71.4% of the original level in the controls at the time of the second measurement, whereas in mice receiving 25 or 50 mg/kg of PTT-01 the mobility had fallen to 31.9% and 23.1% respectively of the base-line values.

(c) Rotating bar test

Mice were trained to remain on a rotating bar for at least 3 minutes. A group of five of the trained mice were given an intraperitoneal injection of 25 mg/kg of PTT-01 in a 1% suspension of tragacanth gum. Another group of five mice were given an intraperitoneal injection of 50 mg/kg of PTT-01 in a 1% suspension of tragacanth gum. All control animals stayed on the bar for 3 minutes. Only two out of the five treated mice receiving 25 mg/kg succeeded in stayng on the bar for 3 minutes but none of those receiving 50 mg/kg dose were able to remain on the bar for 3 minutes.

TABLE 2- Enhancing effect of PTT-01 (I) on sodium pentobarbital (II)- Induced sleep in the mouse

| Treatment | Dose mg/kg intraperitoneally | Animals under hypnosis/ treated | Duration of hypnosis in minutes * ($m \pm SE$) |
|---|---|---|---|
| II | 40 | 3/4 | 3.25 ± 3.4 |
| I + II | 25 + 40 | 4/4 | 52.5 ± 13 |
| I + II | 50 + 40 | 4/4 | 75.2 ± 21.6 |

* Arithmetic mean. Value zero was assigned to animals not losing the righting reflex

TABLE 3- Influence of PTT-01 on spontaneous mobility in mouse

| Intraperitoneal treatment * | Mean number of crossings (± SE) Before treatment | Mean number of crossings (± SE) After treatment | Percent reduction at 2nd measurement |
|---|---|---|---|
| Saline solution | 270.6±24 | 194.4±23 | 28.6 (5) ** |
| PTT-01, 25 mg/kg | 261.5±25 | 84.1±15 | 68.1 (6) |
| PTT-01, 50 mg/kg | 229 ±15 | 50.5±5 | 76.9 (6) |

* The treatment was given immediately after the first measurement or 10 minutes before the second measurement.

** In parentheses the number of the animal groups ( 4 animals for each group)

IV. Sedative effect (Physiology)

The sedative effect produced by PTT-01 was much more similar to the effect of neuroleptic drugs than to that produced, for instance, by barbituric acid derivatives.

This particular feature of the compound could be deduced from the influence the compound exerted on: (a) d-amphetamine toxicity to mice kept in group, and (b) response time of the avoidance reflex.

(a) Antagonism towards d-amphetamine

Six groups of 4 mice each were treated with 5 mg/kg of d-amphetamine sulfate by intraperitoneal administration. Six groups of 4 animals each received the same dose of d-amphetamine sulfate plus 25 mg/kg of PTT-01 intraperitoneally, 15 minutes after d-amphetamine sulfate; six groups of 4 animals each received saline solution and were used as controls. Spontaneous mobility was observed in every animal according to the procedure described hereinbefore. The number of times each animal crossed the light beam throughout the 10 minutes test was noted. As appears from Table 4, the treatment with PTT-01 did not prevent d-amphetamine from stimulating spontaneous mobility.

However, PTT-01 reduced mortality from d-amphetamine. The same amount of PTT-01 was injected intraperitoneally. Then, 10 minutes later, an intraperitoneal injection of 10 mg/kg of d-amphetamine sulfate was administered. Eight animals were used in this test and kept in a 25 x 20 x 14 cm cage in an air-conditioned room (20°C, 60% humidity). All the eight controls died, whereas only 5 of the eight animals treated with PTT-01 died. PTT-01 protected the animals from the effects of d-amphetamine and a remarkable reduction of mortality was obtained.

(b) Avoidance reflex

A conditioning cage consisting of two chambers was used, with free access between the chambers. The floor of one compartment was electrified to make it possible to deliver a mild shock. Mice were conditioned by first giving a warning signal and then, after a short period of time the shock was applied. Mice learned to leave the electrified compartment after the warning bell sounded were then judged to be conditioned. The average period of time, in this experiment, for controls (4) within which they leave the compartment was 53.3±4.5 sec. Four conditioned mice were given PTT-01 intraperitoneally (25 mg/kg); time within which they left the compartment was 60.6 ± 3.9 sec. Four conditioned mice treated with 50 mg/kg of PTT-01 left the compartment only after 77.5 ± 2.7 sec.

V. Anti-convulsant effect

The anti-convulsant effect was determined by the extent to which PTT-01 antagonized the convulsant effect produced by strychnine sulfate or by electroshock. A marked protective effect was observed in both methods.

(a) Antagonism towards strychnine

This type of antagonism was evaluated in the mouse as follows: (1) protection from death caused by a hypodermic injection of strychnine; (2) evaluation of the $LD_{50}$ of strychnine both in mice treated and untreated with PTT-01 and (3) influence on the time required to cause animals to die by slow intravenous perfusion of strychnine.

Details of the experiments and results obtained were as follows: (1) single oral 50 mg/kg dose of PTT-01 protected all the animals against the lethal effect of 1.2 mg/kg of strichnine sulfate in mouse. Groups of six mice were used. Results are given in Table 5.

(2) increasing amounts of strychnine sulfate were injected hypodermically. In two experiments 20 or 40 mg/kg of PTT-01 were given by intraperitoneal route 30 minutes before the strichnine injection and the $LD_{50}$ was then calculated. The following results were obtained (95% confidence limits):

| | $LD_{50}$, mg/kg of strychnine |
|---|---|
| Control experiments | 0.83 (1.02-0.67) |
| Pre-treatment with 20 mg/kg of PTT-01 | 1.30 (1.36-1.03) |
| Pre-treatment with 40 mg/kg of PTT-01 | 1.60 (1.74-1.46) |

(3) continuous intravenous infusion at constant rate (0.4 ml/min) of a strychnine sulfate solution (26.66 mg/l) was carried out by injection in a tail vein in mice until they died. One group of animals received 50 mg/kg of PTT-01 as preliminary treatment 15 minutes before the beginning of the infusion. The mean infusion time to cause six controls to die was 134 $\pm$ 3.2 seconds (mean $\pm$ SE). The mean infusion time required to obtain the same effect in animals pretreated with PTT-01 was 210 $\pm$ 6.1 seconds.

In another experiment the infusion rate of strychnine was lowered to 0.25 ml/min. Under this conditions, the mean infusion time was 246 $\pm$ 3.1 seconds in the case of controls (six animals) and 336 $\pm$ 24.3 secondss in treated animals.

When the above mentioned infusion rate was maintained, while reducing the amount of PTT-01 to 25 mg/kg, the mean infusion time was 260 $\pm$ 13.4 seconds in controls and 360 $\pm$ 21.2 seconds in the treated animals.

(b) Antagonism towards electroshock in mouse

Electroshock was produced in mice by applying ear electrodes. Stimulation parameters which could induce the maximal electroshok (MES) were adopted. This was obtained by applying rectangular input shocks for 0.6 seconds. Each input lasted 0.4 minutes and the input frequency was 25/sec. Phenobarbital sodium was used as control substance. It was injected intraperitoneally 10 minutes before the MES. An amount of 25 mg/kg of PTT-01 was injected by the same route as phenobarbital sodium.

As shown by Table 6, PTT-01 was effective as phenobarbital sodium.

TABLE 4- Influence of PTT-01 on amphetamine-induced hypermobility

| Intraperitoneal treatment | Mean number of crossings ($\pm$ SE) in 10 minutes | Number of groups * |
|---|---|---|
| Saline solution | 229.8 $\pm$ 19 | 6 |
| d-amphetamine, 5 mg/kg | 657 $\pm$ 59 | 6 |
| Saline + saline | 252.6 $\pm$ 13 | 6 |
| d-amphetamine + PTT-01 ** | 836 $\pm$ 100 | 6 |

* Number of groups ( 4 animals for each group)

** PTT-01 ( or saline to controls) was given 15 minutes after d-amphetamine injection

TABLE 5- Protective effect of PTT-01 against strychnine

| Pre-treatment * | Dead / treated | Animals with convulsions/treated | Time of death ** in minutes |
|---|---|---|---|
| None | 9/10 | 9/10 | 6 - 15 |
| PTT-01, 50 mg/kg per os | 0/9 | 5/9 | No death |
| None | 8/8 | 8/8 | 7 - 12 |
| PTT-01, 25 mg/kg i.p. | 1/7 | 7/7 | 33 |
| PTT-01, 50 mg/kg i.p. | 0/8 | 3/8 | No death |

* Given 30 minutes before strychnine

** Extreme values

TABLE 6- Protective effect of PTT-01 against electroshock

| Treatment * | mg/kg | Number of animals with | | |
|---|---|---|---|---|
| | | Clonic convulsions | Tonic convulsions | Dead |
| Saline solution | 1000 | 16 (16) ** | 16 (16) | 6/16 |
| Phenobarbital Na | 40 | 8 (8) | 0 (8) | 0/8 |
| PTT-01 | 25 | 8 (8) | 0 (8) | 0/8 |

* 10 minutes before electroshock

** In parentheses the number of treated animal

VI. Influence on the electromyogram

Intraperitoneal injection of 50 mg/kg of PTT-01 caused the e= lectromyographic tracing to become flat in rabbit with electrodes implanted in its cerebral cortex. The effect was similar to the effect caused by 30 mg/kg of intravenous phenobarbital sodium.

In the unrestrained cat with permanent electrodes implanted in the geniculate body, hippocampus, and cerebral cortex, the con= comitant recording of eye movements and neck muscle contractions after doses of 50 or 100 mg/kg of PTT-01 showed that sedation oc= curred without electromyographic alterations which usually indica= te sleep.

In cats conditioned to sleep for 8 hours from late morning, 25 and 35 mg/kg of PTT-01 did not modify the total sleeping time, but reduced the number of cases of REM sleep and the overall length of the period of time spent by the animal in sleeping did not chan ge, the period of time during which the animal sleep was charac= terized by slow waves was comparatively longer.

VII. Pharmacological activity on systems other than the CNS

The effect of a single 50 mg/kg oral dose of PTT-01 was deter mined on (a) diuresis in water-loaded rats, (b) choleresis, (c) ex cretion rate of intravenously injected bromosulphonphthalein in bi le, (d) arterial blood pressure. No treatment caused any signi= ficant change of the above mentioned parameters except that the ra te of urine excretion was slowed in water-loaded rats treated with a daily oral dose of 50 mg/kg of PTT-01, for five subsequent days.

A further object of the present invention are the pharmaceu= tical compositions containing as active ingredient a compound com= prised in the general formula (I) together with pharmaceutically acceptable excipients.

The finished pharmaceutical forms suitable for the oral admi=

nistration can be capsules, tablets, suspensions in water or suitable syrup.

The following examples are given to illustrate the present invention without limiting it in any way.

EXAMPLE 1

Preparation of PTT-01

130 g of α-amino-γ-butyrolactone hydrobromide prepared according to Livak et al. (J.Am. Chem. Soc. 67( 1945), 2218) are dissolved in 1500 ml of pyridine.

The obtained mixture was cooled to 0°C in a ice and water bath and 100 g of the n.butyric acid chloride were added under stirring.

The mixture was stirred at 0°C for 2 hours and then it was heated to 20-22°C and kept at this temperature for about 6 hrs.

The thus obtained reaction mixture was concentrated under reduced pressure to about 400 ml and then was cooled to 2-3°C, thus obtaining brilliant, needele-shaped, white crystals. After filtration and drying, 119.6 g of product were obtained. m.p. 78-79.5 °C Titre 99.2%. The yield, calculated on α-amino-γ-butyrolactone hydrobromide is about 90%.

EXAMPLE 2

Example 1 was repeated by working according to the same procedure, but n.butyric acid chloride was replaced by isobutyric acid chloride.

α-isobutylcarbonyl-amino-γ-butyrolactone was obtained with satisfactory yield. m.p. 88-89 °C.

The compound was administered to laboratory animals at a dose of about 50 mg/kg. The animals were sedated.

EXAMPLE 3

Example 1 is repeated in all essential details, except that n.bu=

tyric acid chloride is replaced by pentanoic acid chloride. A good yield of α-n.pentylcarbonyl-amino-γ-butyrolactone is obtained. The compound was administered to laboratory animals at a dose of about 50 mg/kg. The animals were sedated.

EXAMPLE 4

Preparation of the compound 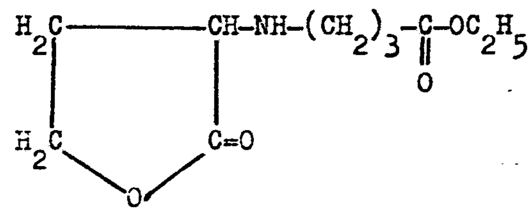

165 g (1 mole) of α-bromine-butyrolactone were dissolved in 1500 ml of pyridine, at room temperature and under stirring. The thus obtained solution was added, portionwise and within about 30 minutes, with 131 g (1 mole) of the ethyl ester of the γ-amino-butyric acid. The mixture was slightly boiled for 3 hrs. under stirring. Pyridine was distilled preferably under vacuum and after having reduced the volume of the solution to about 1/3, the solution was allowed to cool to room temperature. The solution was poured into a triple volume of cold water, under stirring. After having allowed the solution to stand overnight, the obtained precipitate was filtered. White, bright flakes were dried in vacuo, at 40-50°C, and recrystallized from ethyl alcohol. The yield of the crystallized product, calculated on the starting bromine-butyrolactone, was 78-80%. m.p. 150-160°C ( decomposition).

## CLAIMS

1. Derivatives of α-amino-γ-butyrolactone endowed with anti-convulsant, anti-epileptic, sedative action and useful for inhibiting ingestion of alcohol and intoxicating drinks, comprised in the general formula (I):

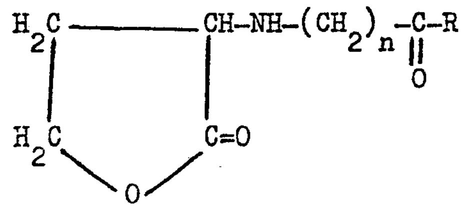

(I)

( wherein R is a linear or branched alkyl radical containing from 4 to 8 carbon atoms; or R is -OR', R' being a linear or branched alkyl radical containing from 1 to 5 C; n is always zero when R is alkyl, or n is an integer different from zero and comprised between 1 and 5 when R is -OR') and their pharmaceutically acceptable salts.

2. α-n. butylcarbonyl-amino-γ-butyrolactone

3. α-isobutylcarbonyl-amino-γ-butyrolactone

4. α-pentylcarbonyl-amino-γ-butyrolactone

5. The compound 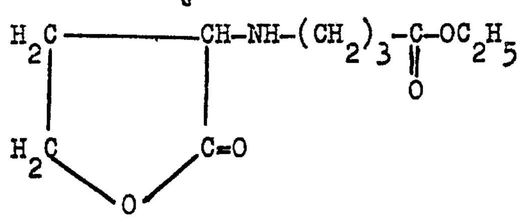

6. Method for the preparation of the compounds of the general formula (I) wherein R is a linear or branched alkyl radical containing from 4 to 8 C, and n is zero, characterized by the fact that the corresponding acid chloride is allowed to react with the selected α-amino-γ-butyrolactone, at reduced temperatures, in the presence of an organic base.

7. Method for the preparation of the compounds of the general formula (I) wherein R is -OR', R' being a linear or branched alkyl

radical containing 1-5 C and n, always different from zero, is an integer from 1 to 5, characterized in that the ester of a ω-aminoacid is allowed to react with the α-halo-γ-butyrolactone.

8. Pharmaceutical compositions containing as active ingredient a compound of the general formula (I)

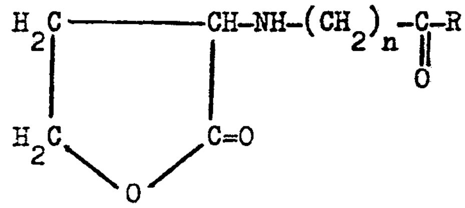

(I)

( wherein R and n have the above mentioned meanings), or a pharmaceutically acceptable salt thereof, together with pharmaceutically acceptable excipients.

9. Pharmaceutical compositions containing as active ingredient α-n.butylcarbonyl-amino-γ-butyrolactone, together with pharmaceutically acceptable excipients.